# EUROPEAN PATENT APPLICATION

(11) **EP 1 609 421 A1**
(43) Date of publication of application: **28.12.2005**
(21) Application number: 05253590.3
(22) Date of filing: 10.06.2005
(51) Int. Cl.: A61B 8/00

(54) **Methods and apparatus for defining a protocol for ultrasound machine**

(30) Priority: 22.06.2004 US 581675; 29.06.2004 US 583578; 26.08.2004 US 926754
(71) Applicant: GENERAL ELECTRIC COMPANY, Schenectady, NY 12345 (US)
(72) Inventor: Lundberg, Vidar, 7024 Trondheim (NO); Maehle, Jorgen, 7042 Trondheim (NO); Steen, Erik Norman, 1517 Moss (NO); Stavo, Arve, 7024 Trondheim (NO)
(74) Representative: Pedder, James Cuthbert

(57) **Abstract**

A protocol-based ultrasound system (100) is described comprising a memory (136) storing a template (140) comprised of cells, each of the cells containing scan parameters (138) defining a scan sequence for acquisition of ultrasound images along at least two scan planes trough an object. The system includes a user input (134) for entering, prior to scanning the object, parameter values for the scan parameters (138) associated with the cells in the template (140) to define the scan sequences along the at least two scan planes. Also included is a probe (106) scanning the object to automatically and successively acquire ultrasound images along the at least two scan planes based on the parameter values for the scan parameters (138) in the cells.

## Description

The present invention relates to diagnostic ultrasound methods and systems. In particular, the present invention relates to methods and apparatus for defining a protocol in accordance with which ultrasound scans are automatically performed.

Numerous ultrasound methods and systems exist for use in medical diagnostics. Various features have been proposed to facilitate patient examination and diagnosis based on ultrasound images of the patient. For example, in stress-echo type heart studies, portions of the heart may be scanned before and after a stress test to provide corresponding base-line and stress-level images of the selected portions of the heart.

However, scanning the same portions of the heart during the base-line and stress levels of the tests is difficult using current technology. The operator may have difficulty in capturing the same portion of the heart repeatedly during the stress test because, for example, the patient is breathing harder at each stress level and because the heart is beating faster and moving to a greater extent within the patient's body than during the base-line acquisition. The operator may have difficulty positioning the probe to obtain the same reference views and angles of the scanned object at advanced stress levels as before stress for base-line recording. The base-line and stress-level image slices may not show the same portions and/or views of the heart such that a physician may have to mentally visualize the anatomy based on the differing 2D scans and correct for the differences between the before and after slices.

Further, the user normally obtains multiple ultrasound images while the patient is in a base-line state and an equal number of ultrasound images at each stress level. At base-line and at each stress level, the ultrasound images are recorded continuously to form a cine loop of real-time images of the myocardium along a select scan plane. Also at each base-line and stress level, cine loops of ultrasound images are acquired along multiple scan planes.

Heretofore, the user manually adjusted a series of scan parameters between acquisition along each scan plane. The manual adjustment process was repeated for each scan plane at base-line and at each stress level. This process was slow and awkward for users and delayed completion of a stress test.

A need exists for methods and systems that are able to define a protocol for automated acquisition of ultrasound images along multiple scan planes.

A protocol-based ultrasound method is provided according to the invention. The ultrasound method provides a template comprised of cells. Each of the cells contain scan parameters defining a scan sequence for acquisition of ultrasound images along scan planes through an object. Prior to scanning the object, parameter values for the scan parameters associated with cells in the template are entered by the user to define the scan sequences along at least two scan planes. The object is scanned with an ultrasound probe to automatically and successively acquire ultrasound images along at least two scan planes based on the parameter values for the scan parameters in the cells.

In accordance with an alternative embodiment, a method displays ultrasound images of an object that is changing states in accordance with a protocol. Each of the ultrasound images is acquired along a corresponding scan plane through the object. A collection of ultrasound images is provided. Each of the ultrasound images is acquired along an associated scan plane while the object is in an associated state. The display is segmented into at least two quadrants or regions. A corresponding ultrasound image is presented in the quadrants, wherein co-displayed ultrasound images correspond to one of a common state of the object and a common scan plane through the object.

In accordance with yet another embodiment, an ultrasound system is provided with memory for storing a template comprised of cells. Each of the cells contains parameters defining acquisition for an ultrasound image along a corresponding scan plane through an object. The configuration includes an input for entering, prior to scanning the object, parameter values for the scan parameters associated with the cells in the template. The system includes a probe for scanning the object to automatically acquire ultrasound images along at least two scan planes based on the parameter values for the scan parameters in the cells.

The invention will now be described in greater detail, by way of example, with reference to the drawings, in which:-
Figure 1 is a block diagram of an ultrasound system formed in accordance with an embodiment of the present invention.
Figure 2 is a block diagram of an ultrasound system formed in accordance with an embodiment of the present invention.
Figure 3 is a flowchart of an exemplary method for use of a protocol template in performing stress scans.
Figure 4 illustrates a screen display divided into four quadrants in accordance with an embodiment of the present invention.
Figure 5 illustrates a template of cells in accordance with an embodiment of the present invention.
Figure 6 illustrates a screen display divided into four quadrants in accordance with an embodiment of the present invention.
Figure 7 illustrates a screen display that displays volumetric 3D/4D stress echo scans in accordance with an embodiment of the present invention.
Figure 8 illustrates a portion of a template formed in accordance with an embodiment of the present invention.
Figure 9 illustrates a storage format for organizing ultrasound images taken during a patient examination formed in accordance with an embodiment of the present invention.

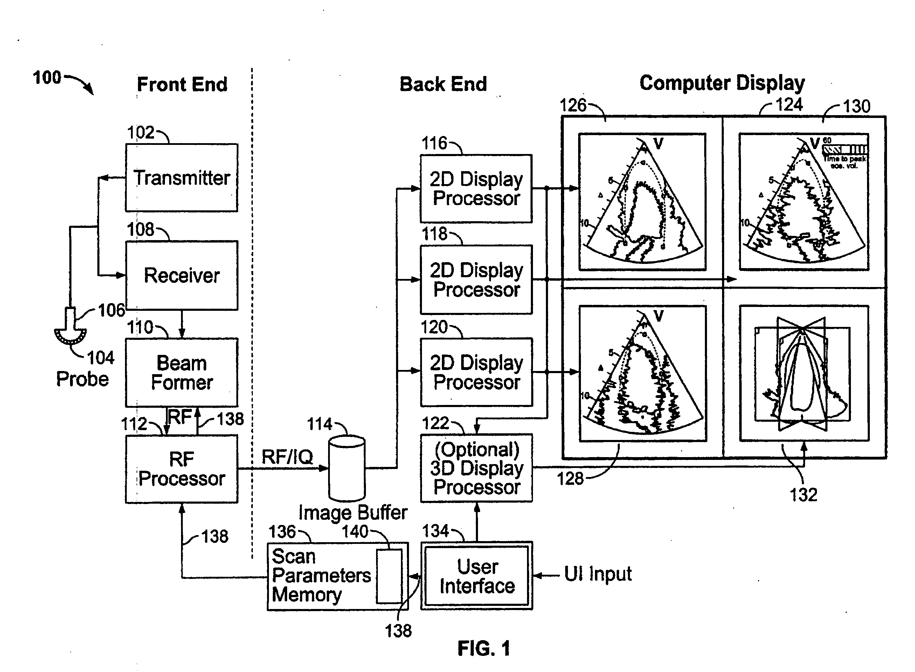

Figure 1 is a block diagram of an ultrasound system 100 formed in accordance with an embodiment of the present invention. The ultrasound system 100 is configurable to acquire ultrasound information corresponding to a plurality of two-dimensional (2D) representations or images of a region of interest (ROI) in a subject or patient. One such ROI may be the human heart or the myocardium of a human heart. The ultrasound system 100 is configurable to acquire 2D image planes in two or three different planes of orientation. The ultrasound system 100 includes a transmitter 102 that, under the guidance of a beamformer 110, drives a plurality of transducer elements 104 within an array transducer 106 to emit pulsed ultrasound signals into a body. The elements 104 within the array transducer 106 are excited by an excitation signal received from the transmitter 102 based on control information received from the beamformer 110.

When excited, the transducer elements 104 produce ultrasonic waveforms that are directed along transmit beams into the subject. The ultrasound waves are back-scattered from density interfaces and/or structures in the body, like blood cells or muscular tissue, to produce echoes which return to the transducer elements 104. The echo information is received and converted into electrical signals by the transducer elements 104. The electrical signals are transmitted by the array transducer 106 to a receiver 108 and subsequently passed to the beamformer 110. In the embodiment described below, the beamformer 110 operates as a transmit and receive beamformer.

The beamformer 110 receives and uses scan parameters 138 to control the operation of the transmitter 102 to generate ultrasound scan beams and the receiver 108 to collect echo information. The beam former through the use of information provided in the scan parameters 138 produces received beam information that may be used in generating ultrasound images. Beamformer 110 delays, apodizes and sums each electrical signal with other electrical signals received from the array transducer 106. The summed signals represent echoes from the ultrasound beams or lines. The summed signals are output from the beamformer 110 to an RF processor 112. The RF processor 112 may generate in phase and quadrature (I and Q) information. Alternatively, real value signals may be generated from the information received from the beamformer 110. The RF processor 112 gathers information (e.g. I/Q information) related to one frame and stores the frame information with time stamp and orientation/rotation information into an image buffer 114. Orientation/rotation information may indicate the angular rotation one frame makes with another. For example, in a tri-plane situation whereby ultrasound information is acquired simultaneously for three differently oriented planes or views, one frame may be associated with an angle of 0 degrees, another with an angle of 60 degrees, and a third with an angle of 120 degrees. Thus, frames may be added to the image buffer 114 in a repeating order of 0 degrees, 60 degrees, 120 degrees, ...0 degrees, 60 degrees, 120 degrees, .... The first and fourth frame in the image buffer 114 have a first common planar orientation. The second and fifth frames have a second common planar orientation and the third and sixth frames have a third common planar orientation.

Alternatively, in a biplane situation, the RF processor 112 may collect frame information and store the information in a repeating frame orientation order of 0 degrees, 90 degrees, 0 degrees, 90 degrees, etc. The frames of information stored in the image buffer 114 are processed by the 2D display processor 116.

The 2D display processors 116, 118, and 120 operate alternatively and successfully in round-robin fashion processing image frames from the image buffer 114. For example, the display processors 116, 118, and 120 may have access to all of the data slices in the image buffer 114, but are configured to operate upon data slices having one angular orientation. For example, the display processor 116 may only process image frames from the image buffer 114 associated with an angular rotation of 0 degrees. Likewise, the display processor 118 may only process 60 degree oriented frames and the display processor 120 may only process 120 degree oriented frames.

The 2D display processor 116 may process a set of frames having a common orientation from the image buffer 114 to produce a 2D image or view of the scanned object in a quadrant 126 of a computer display 124. The sequence of image frames played in the quadrant 126 may form a cine loop. Likewise, the display processor 118 may process a set of frames from the image buffer 114 having a common orientation to produce a second different 2D view of the scanned object in a quadrant 130. The display processor 120 may process a set of frames having a common orientation from the image buffer 114 to produce a third different 2D view of the scanned object in a quadrant 128.

For example, the frames processed by the display processor 116 may produce an apical 4-chamber view of the heart to be shown in the quadrant 126. Frames processed by the display processor 118 may produce an apical 2-chamber view of the heart to be shown in the quadrant 130. The display processor 120 may produce frames to form an apical long-axis (APLAX) view of the heart to be shown in the quadrant 128. All three views of the human heart may be shown simultaneously in real time in the three quadrants 126, 128, and 130 of the computer display 124.

A 2D display processor, for example the processor 116, may perform filtering of the frame information received from the image buffer 114, as well as processing of the frame information, to produce a processed image frame. Some forms of processed image frames may be B-mode data (e.g. echo signal intensity or amplitude) or Doppler data. Examples of Doppler data include color Doppler velocity data (CDV), color Doppler energy data (CDE), or Doppler Tissue data (DTI)). The display processor 116 may then perform scan conversion to map data from a polar to Cartesian coordinate system for display on a computer display 124.

Optionally, a 3D display processor 122 may be provided to process the outputs from the other 2D display processors 116, 118, and 120. Processor 122 may combine the 3 views produced from 2D display processors 116, 118, and 120 to form a tri-plane view in a quadrant 132 of the computer display 124. The tri-plane view may show a 3D image, e.g. a 3D image of the human heart, aligned with respect to the 3 intersecting planes of the tri-plane. In one embodiment, the 3 planes of the tri-plane intersect at a common axis of rotation. In other embodiments, any number of planes may have any orientation. For example, the user may want to acquire a number of short-axis scan planes simultaneously from the parasternal window at different levels from apex to mitral plane in the heart. In this case, N number of planes are acquired with same rotation angle but different tilt angle.

A user interface 134 is provided which allows the user to input scan parameters 138. The scan parameters 138 are associated with the cells of a template 140 stored in a memory 136. The scan parameters 138 may allow the user to designate whether a biplane or tri-plane scan is desired, and rotation and tilt angles between planes. The scan parameters 138 may allow for adjusting the depth and width of a scan of the object for each of the planes of the biplane or tri-plane. The scan parameters 138 may include parameters for gain, frequency, focus position, mode, contact, and zoom. When performing simultaneous acquisition of scan data from three planes of a tri-plane, the beamformer 110 in conjunction with the transmitter 102 signals the array transducer 106 to produce ultrasound beams that are focused within and adjacent to the three planes that slice the scan object. The reflected ultrasound echoes are gathered simultaneously to produce image frames that are stored in the image buffer 114. As the image buffer 114 is being filled by the RF processor 112, the image buffer 114 is being emptied by the 2D display processors 116, 118, and 120. The 2D display processors 116, 118, and 120 form the data for viewing as 3 views of the scan object in corresponding computer display quadrants 126, 130, and 128. The display of the 3 views in quadrants 126, 130, and 128, as well as an optional displaying of the combination of the 3 views in quadrant 132, is in real time. Real time display makes use of the scan data as soon as the data is available for display.

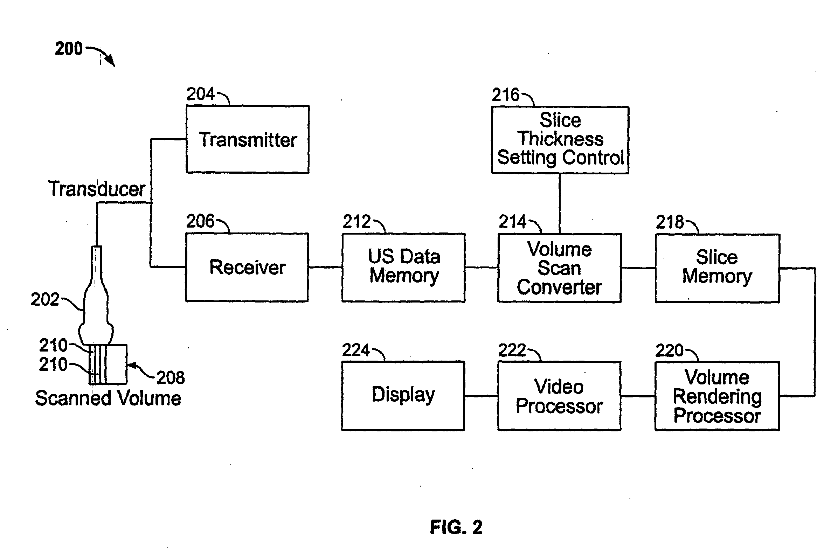

Figure 2 is a block diagram of an ultrasound system 200 formed in accordance with an embodiment of the present invention. The system includes a probe 202 connected to a transmitter 204 and a receiver 206. The probe 202 transmits ultrasonic pulses and receives echoes from structures inside of a scanned ultrasound volume 208. The memory 212 stores ultrasound data from the receiver 206 derived from the scanned ultrasound volume 208. The volume 208 may be obtained by various techniques (e.g., 3D scanning, real-time 3D imaging, volume scanning, 2D scanning with transducers having positioning sensors, freehand scanning using a voxel correlation technique, 2D or matrix array transducers and the like).

The probe 202 is moved, such as along a linear or arcuate path, while scanning a region of interest (ROI). At each linear or arcuate position, the probe 202 obtains scan planes 210. Alternatively, a matrix array transducer probe 202 with electronic beam steering may be used to obtain the scan planes 210 without moving the probe 202. The scan planes 210 are collected for a thickness, such as from a group or set of adjacent scan planes 210. The scan planes 210 are stored in the memory 212, and then passed to a volume scan converter 214. In some embodiments, the probe 202 may obtain lines instead of the scan planes 210, and the memory 212 may store lines obtained by the probe 202 rather than the scan planes 210. The volume scan converter 214 may process lines obtained by the probe 202 rather than the scan planes 210. The volume scan converter 214 receives a slice thickness setting from a control input 216, which identifies the thickness of a slice to be created from the scan planes 210. The volume scan converter 214 creates a 2D frame from multiple adjacent scan planes 210. The frame is stored in slice memory 218 and is accessed by a volume rendering processor 220. The volume rendering processor 220 performs volume rendering upon the frame at a point in time by performing an interpolation of the values of adjacent frames. The output of the volume rendering processor 220 is passed to the video processor 222 and the display 224.

The position of each echo signal sample (voxel) is defined in terms of geometrical accuracy (i.e., the distance from one voxel to the next) and ultrasonic response (and derived values from the ultrasonic response). Suitable ultrasonic responses include gray scale values, color flow values, and angio or power Doppler information. Power Doppler information is not suitable for surface rendering of quantitative information. Surface rendering of quantitative information requires the aquisition of a B mode (or gray scale) slice. Interpolation of adjacent frames or planes at different depths is performed in first and second scan planes that intersect with one another along a common axis to derive synthetic ultrasound data estimating a surface of the object.

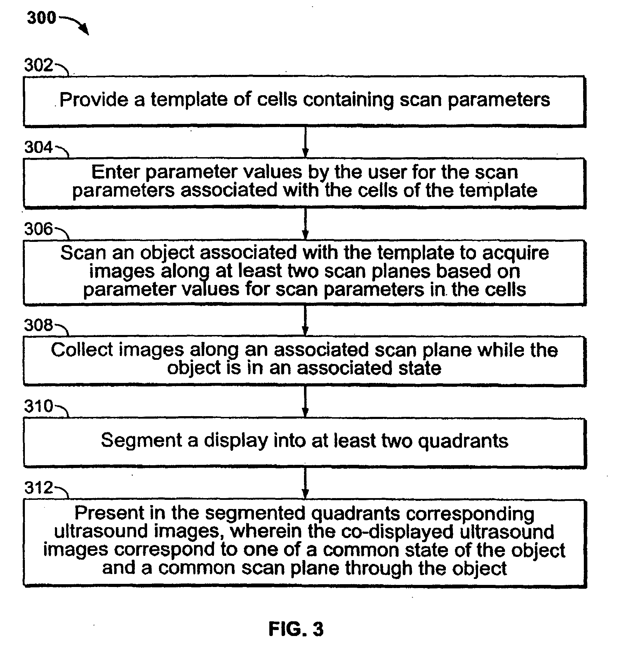

Figure 3 is a flowchart 300 of an exemplary method for use of a protocol defined in a template for performing stress echo examination of a patient. The method provides at 302 a template comprised of cells. Each cell contains scan parameters defining acquisition of data along a corresponding scan plane through an object. The object may be a patient. The ultrasound system 100 acquires one or a series of ultrasound images along each scan plane based on the associated values for the scan parameters. The type of multiplane acquisition, e.g. biplane, tri-plane, or N-plane, may be specified in a cell of the template by the user. The system 100 sets the angles, orientation, tilt, and the like for a given type of multiplane scan for the planes with respect to one another based on predefined default values or user entered parameter values. For example, if the user designates tri-plane imaging for the template, the user may then set parameter values for the angles of the planes to be 0 degrees, 60 degrees and 120 degrees with respect to a base reference plane. The system 100 uses the user entered parameters values during a base-line examination, and the same parameter values are remembered and used by the system 100 during examination at each stress level in the test. In one example, the template corresponds to a stress echo examination for a patient and each of the cells corresponds to one of a base line and discrete stress levels.

Prior to scanning the object, parameter values may be entered by the user at 304 for the scan parameters associated with cells in the template defining the protocol. Generic scan parameters may be set before the patient arrives for the exam, such as based on the type of stress exams to be performed. The scan parameters may include patient-specific scan parameters in addition to generic scan parameters, which are set when the patient arrives for the examination. For example, if the dimensions of a patient's heart are larger or smaller than normal, the patient-specific scan parameters may need to be adjusted accordingly.

The parameter values for all of the cells in the template are entered by a user before beginning any portion of the complete examination. For example, the second and third cells may specify scan parameters for the plane angles, tilt and the like that are set to default levels or by the user based on the type of multiplane scan that may be specified in the first cell.

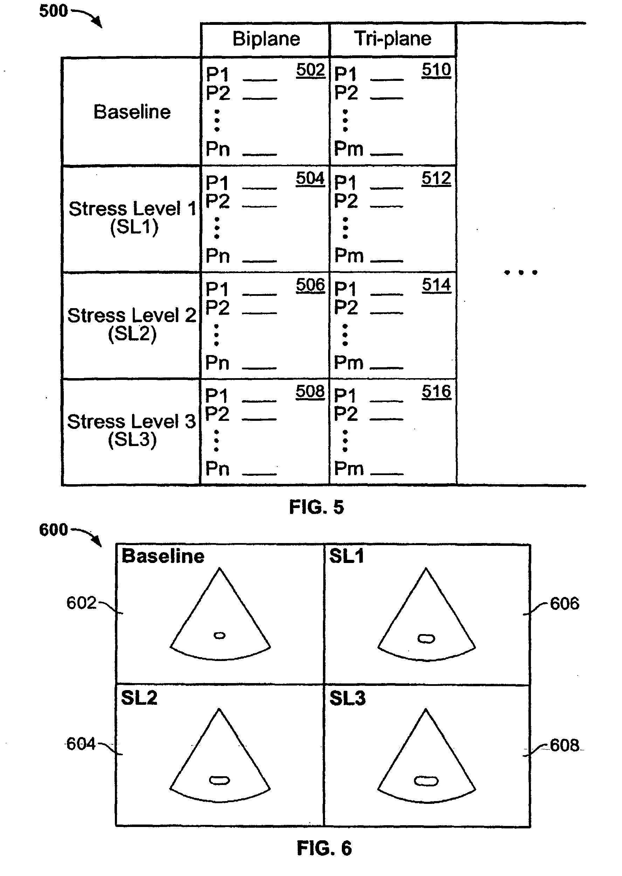

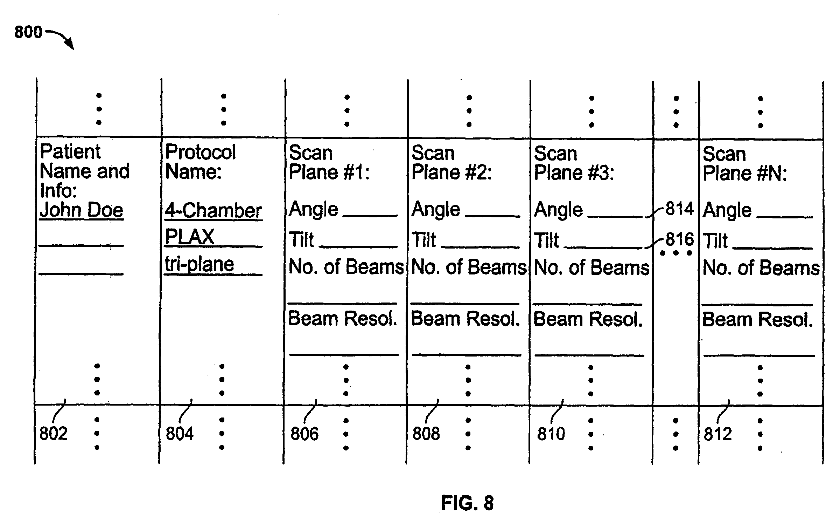

The scan parameters include protocol generic parameters. The scan parameters may define three scan planes intersecting along a common axis extending from the probe. The object is scanned substantially simultaneously along the three scan planes based on the parameter values of an associated cell. Alternatively, the scan parameters may define two scan planes intersecting along a common axis extending from the probe. The object is scanned substantially simultaneously along the two scan planes based on the parameter values of an associated cell. In general, the scan parameters may define multiple planes that are scanned along based on the parameter values of an associated cell. Examples of a template format of cells that may be used to define scan protocols are provided in Figures 5 and 8.

Figure 5 illustrates a template 500 of cells 502-516 in accordance with an embodiment of the present invention. In the embodiment exemplified by Figure 5, a cell contains information about all the planes for a recording, e.g. a biplane or a tri-plane recording. A cine recording may be acquired for all planes specified by the parameter information of a cell and stored into an image file that is associated with the cell. For example, by selecting and populating the cell 502, the user has selected for the ultrasound system 100 (Figure 1) to perform some type of biplane scan, e.g. a PLAX-PSAX scan, for the patient. By selecting and populating the cell 510, the user has selected to have the ultrasound system 100 perform a tri-plane scan for the patient. Parameters P1 and P2 of cell 502 may correspond to the scan width and depth of the scan, and may require valuing by the user. Other parameters may correspond to the gain, frequency and focus depths. Once parameters within the cell 502 that require valuing by the user are valued, the template may automatically populate values for the remaining parameters of the cell 502 based on the values entered for required parameters. The cells 504, 506, and 508 correspond to scan parameters for defining a stress level 1 (SL1), stress level 2 (SL2), and stress level 3 (SL3) acquisition of data. The parameters of cells 504, 506, and 508 may be automatically populated from the parameters of cell 502, in which case the scan data is collected in a similar fashion as the data for cell 502. Differences in the images produced for base-line and SL1-SL3 occur due to the inducement of the different stress levels in the patient.

The user may select and populate cells 502 and 510 of the template 500 to designate acquiring scan data for both biplane and tri-plane recordings. In selecting and entering parameter information for both columns (biplane and tri-plane) of the template 500, the system 100 of Figure 1 acquires scan data for both a biplane and tri-plane view. The biplane and tri-plane acquisitions in one stress level may be done as follows, for example. First a biplane acquisition from the parasternal window (trans-thoracic window) is performed to get PLAX and PSAX projections, then the probe 106 (Figure 1) is moved to perform the tri-plane acquisition from the apical window (trans-thoracic window) to get 4-ch, 2-ch and APLAX projections.

Figure 8 illustrates a portion of a template 800 formed in accordance with an embodiment of the present invention. The embodiment exemplified by Figure 8 is different from that exemplified by Figure 5 in that multiple cells contain the parameter information for the planes of a recording. For example, a biplane recording may populate cells 802, 804, 806, and 808 in Figure 8, while in only the cell 502 is populated in Figure 5 to specify a biplane recording. The template 800 shows a row of cells 802, 804, 806, 808, 810, and 812 that define for a patient an ultrasound test to be performed on the patient. The values for the row of cells 802-812 may be used for a base-line test and remembered by the system 100 (Figure 1) for successive stress-level tests. The cell 802 is valued with the patient's name and information. The valuing or population of values for the cells 802-812 may be done before the patient arrives for testing. The cell 804 identifies the protocol name or test type to be performed on the patient, which may also include or determine the number of scan planes along which to collect scan information. The number of scan planes may determine the number of succeeding cells in the row needing valuing. The cell 806 defines scan parameter information for a scan plane #1. The cell 808 defines scan parameter information for a scan plane #2, and the cell 810 for a scan plane #3. An angle 814 and a tilt 816 are examples of scan parameters. As an example, the cell 806 may have the angle 814 valued with 0 degrees and the tilt 816 valued with 0 degrees. The cell 808 may have the angle 814 valued with 60 degrees and tilt 816 valued with 0 degrees. The cell 810 may have the angle 814 valued with 120 degrees and the tilt 816 valued with 10 degrees. A template 800 may be provided with a default number of columns or cells in a row, e.g. the cell 812 being for an Nth scan plane.

Some scan parameters may be automatically valued with normally preferred default values as the user enters values for the scan parameters. For example, the user may value the cell 804 with a protocol name indicating that a 4-chamber PLAX tri-plane scan test is needed. The system 100 may then value the angle 814 and the tilt 816 of cells 806, 808, and 810 with appropriate default values for a patient with a normal heart size and shape. If after the patient arrives for testing, the patient's heart size and/or shape are not normal, the user may adjust the values for the angle 814 and the tilt 816 parameters accordingly. Such changes to the scan parameter values may be remembered by the system 100 for performing successive stress tests. In an alternative embodiment, selecting the name of a column in the template (e.g. "Par LAX SAX") will result in default scan parameters for the number of planes and angles (e.g. 2 planes with rotation angle 90 degrees and tilt angle -5 degrees).

Returning to Figure 3, at 306, the object is scanned with an ultrasound probe to automatically acquire ultrasound images along at least two scan planes based on the parameter values for the scan parameters in the cells. The object is scanned first and second times that are separated by an interval during which a state of the object is changed. The object is scanned at least first and second times substantially simultaneously (e.g., within 0.02 seconds) along first and second scan planes without moving the probe between the at least first and second times and without adjustment by the user of the scan parameter. Series of consecutive ultrasound images acquired along each of the at least two scan planes may be recorded for playback as a cine-loop. The base-line may be recorded for playback as a cine-loop as well as the different stress levels.

Ultrasound images of an object that is changing states in accordance with a protocol are collected at 308. Each of the ultrasound images is acquired along a corresponding scan plane through the object. A collection of ultrasound images is stored in memory 114. Each of the ultrasound images is acquired along an associated scan plane while the object is in an associated state.

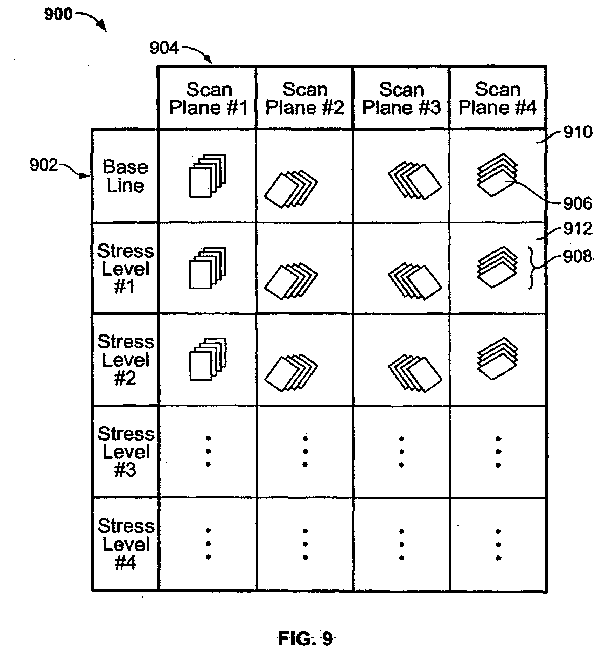

Figure 9 illustrates a storage format 900 for organizing ultrasound images 906 taken during a patient examination. The storage format 900 is an array of cells 910 organized into rows 902 and columns 904. Each column 904 is associated with an acquisition state defined by scan parameters, e.g. scan plane #1, scan plane #2, scan plane #3, or scan plane #4. Each row 902 is associated with an object or patient state, e.g. base-line, stress level #1, stress level #2, stress level #3, or stress level #4. For example, the cell 910 stores an ultrasound image 906 for a scan acquired along scan plane #4 when the patient is at base-line with no induced stress. A real-time series of ultrasound images forming a cine loop 908 may be stored in a cell 912. The cine loop 908 of the cell 912 provides for a playback of the images acquired along scan plane #4 when the patient is at stress level #1. In an alternative embodiment, the four columns shown in Figure 9 for scan plane #1, scan plane #2, scan plane #3, and scan plane #4 may be collapsed into one column such that a cell is provided for the baseline row. The embodiment in this case may associate the cine loop for all planes for a given object state (e.g. baseline) to one cell.

Returning to Figure 3, at 310, the display is segmented into at least two quadrants. Four quadrants may be shown, and the quadrants may contain ultrasound images acquired along a common scan plane while the object is in four different states.

At 312, corresponding ultrasound images are presented in the quadrants, wherein co-displayed ultrasound images correspond to one of a common state of the object and a common scan plane through the object. Three quadrants, containing ultrasound images acquired at three different scan planes while the object is in a common state may be presented. A recorded series of consecutive ultrasound images may be played back in at least two of the quadrants.

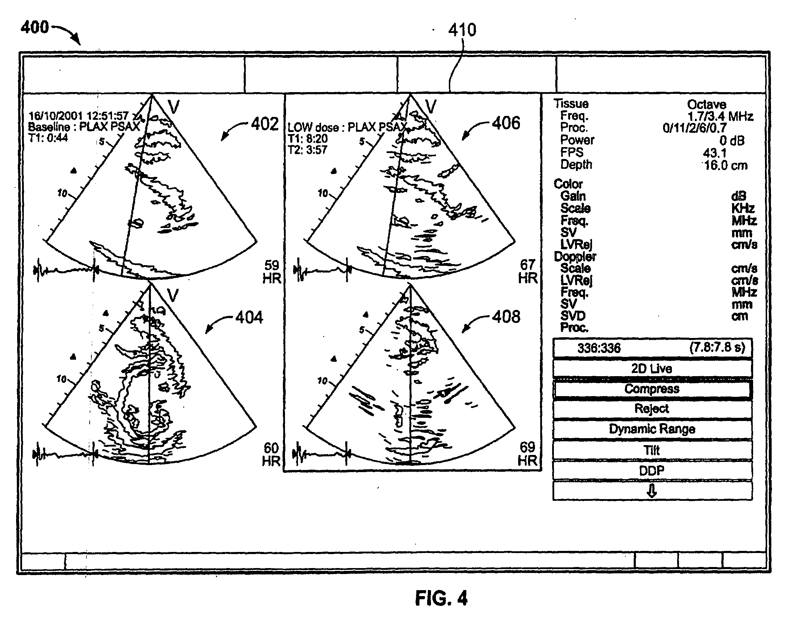

Figure 4 illustrates a screen display 400 divided into four quadrants 402, 404, 406, and 408 in accordance with an embodiment of the present invention. The quadrants 402 and 404 in the left side or column of the screen display 400 show a base-line scan along two planes (biplane scan) of an object, e.g. a patient's heart. Quadrant 402 displays a scan image of the patient's heart along an parasternal long-axis (PLAX) plane, and quadrant 404 displays a scan image of the patient's heart along an parasternal short-axis (PSAX) plane. A series of consecutive ultrasound images may be acquired along each of the two planes to form a cine loop for each of the two planes. The images shown in quadrants 402 and 404 are at base-line. The base-line PLAX and PSAX scans are taken before the patient is submitted to stress. The selected right column 410 of the screen display 400 shows a stress-level scan, e.g. a stress level 1 (SL1) scan, of the patient's heart after the patient has been submitted to stress. The image shown in quadrant 406 is from the same orientation and portion of the heart as the image shown in quadrant 402, except the image of quadrant 406 is after stress has been induced in the patient.

In comparing the views in quadrants 402 and 406, the user is able to see the same area of the heart correspondingly before and after stress inducement. Likewise, quadrants 404 and 408 show a same view of the heart before and after stress inducement. The base-line quadrants 402 and 404 may be a cine loop of images acquired during base-line, while the quadrants 406 and 408 may show live images of the patient after undergoing a stress level 1 (SL1). To acquire or collect 308 the images displayed in the quadrants 402-408, a stress template may be used.. Although Figure 4 shows only the column 410 for display of stress test images, a user may alternate between displaying images acquired for SL1, SL2, and SL3 in the column 410 by sequentially clicking a mouse pointer, for example, in the column 410. Figure 4 illustrates the situation in protocol acquisition (live scanning) when the left side of the screen shows the reference image (base-line) and the right side shows a live image. After acquisition of all stress levels is completed, the user may review a combination of images in protocol analysis, e.g. by selecting images with a mouse cursor, or more typically by recalling a protocol analysis group. A tri-plane view or display is similar to the display of the biplane view in screen display 400, with the addition of a third row in the tri-plane display for a third scan plan.

Figure 6 illustrates a screen display 600 divided into four quadrants 602, 604, 606, and 608 in accordance with an embodiment of the present invention. Each quadrant shows a view along the same scan plane, but at different times, e.g. at base-line, SL1, SL2, and SL3. The screen display 600 provides the user a view of the patient along the same scan plane at different levels of induced stress. The user interface 134 (Figure 1) may provide through use of a mouse, for example, a method for selecting a different scan plane to view in the four quadrants 602-608 that correspond to base-line, SL1, SL2, and SL3. When analyzing a scan plane from four different levels from biplane or tri-plane recordings, a Next button may be implemented in the user interface 134 that sequences through all scan planes in the recordings. If currently seeing the last scan plane and hitting Next, the system may sequence to the next analysis group of recordings..

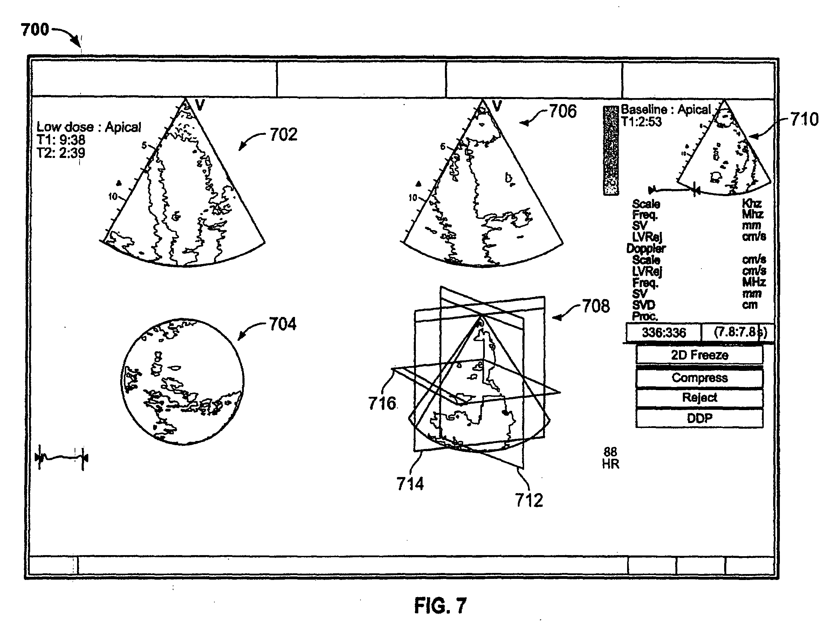

Figure 7 illustrates a screen display 700 that displays volumetric 3D/4D stress echo scans in accordance with an embodiment of the present invention. Figure 7 illustrates an embodiment of a screen display 700 with four quadrants 702, 704, 706, and 708, and a smaller quadrant 710. Typically, one volume is acquired for each stress level, and various views of the volume at a stress level are displayed in the quadrants 702-708 of the screen display 700. In an alternative embodiment, two or more volumes may be acquired for a stress level corresponding to two or more views or probe positions. In the alternative embodiment, each quadrant of a screen display may display a view of a different volume acquired for one or more stress levels. Figure 7 shows a view of the same volume acquired at base-line or at a predetermined stress level in quadrant 710.

The quadrant 710 may display a 2D view of the volume at base-line. A corresponding view may be shown in one of the other quadrants, e.g. the quadrant 702, at a stress level, e.g. at stress level 1 (SL1). The quadrants 704 and 706 may show two different 2D views of the volume at SL1. For example, the view presented in quadrant 704 may be viewing the volume for a short-axis heart view at SL1. The view presented in quadrant 706 may be viewing the volume for a 2-chamber heart view at SL1. The view presented in quadrant 702 may be viewing the volume for a 4-chamber heart view at SL1. Quadrant 708 shows a 3D view from SL1 with at least two cut planes 712 and 714 that indicate the views shown in quadrants 702 and 706. The horizontal cut plane 716 indicates to the user at what depth a short-axis view is shown in quadrant 704. The user via the user interface 134 (Figure 1) may adjust the scan parameters 138 to elevate or lower the horizontal cut plane 716 along the LDV axis to vary the depth of the short-axis view displayed in quadrant 704. In other embodiments of the present invention a short-axis display from each of a number of stress levels may be shown, allowing the user to elevate or lower the cut plane synchronously for all displays (also known as short-axis (SAX) sliding). This may be done either in live scanning or after acquiring all stress levels during the analysis phase of the examination.

Similar to the use of the template 500 for multiplane acquisition stress testing, a template may be provided and populated for the volumetric 3D/4D stress echo scan. After valuing the user required parameters, e.g. patient-specific parameters and selected views to be displayed, generic parameters may be determined automatically, for example, whether ECG stitching is to be used and if so, the number of stitches to be used. Parameters may be automatically populated in the stress-level cells of the template from the baseline cell. The 3D/4D template preserves the same geometric orientation and/or volume geometry used for base-line for acquisition of volumes for stress-level tests. For example, the quadrants 702, 704, and 706 display views at SL1 of a volume acquired with the same geometric orientation and/or volume geometry as used for the volume of the base-line view in quadrant 710. Preserving the base-line volume geometry for use in collecting volumes for stress levels is accomplished by the automatic population of parameters in the template cells for the stress levels. The cut planes 712 and 714 of the reference view in quadrant 708 may be rotated. When rotating the reference view in quadrant 708, the views in quadrants 702, 704, 706, and 710 are correspondingly rotated. Thus, the orientation of the views presented in quadrants 702, 704, 706, and 710 are synchronized to the view orientation presented to the user in the reference view of the quadrant 708.

In an alternative embodiment, a protocol template may have volume (3D/4D), 2D multiplane recordings, and single plane recordings combined. The template may be populated for a patient (generic parameters valued) before the patient's arrival for stress tests that include volume, multiplane and single plane stress tests.

Exemplary embodiments of diagnostic ultrasound systems are described above in detail. The systems are not limited to the specific embodiments described herein, but rather, components of each system may be utilized independently and separately from other components described herein. Each system component can also be used in combination with other system components.

## Claims

1. An ultrasound system (100, 200), comprising:
memory (136) storing ( a template (140) comprised of cells (502-516, 802-812, 910-912), each of said cells (502-516, 802-812, 910-912) containing scan parameters (138) defining a scan sequence for acquisition (308) of ultrasound images (906) along at least two scan planes (210, 712, 714) through an object;
a user input (134) for entering (304), prior to scanning (306) the object, parameter values for said scan parameters (138) associated with said cells (502-516, 802-812, 910-912) in said template (140) to define said scan sequences along the at least two scan planes (210, 712, 714); and
a probe (106, 202) scanning (306) the object to automatically and successively acquire (308) ultrasound images (906) along the at least two scan planes (210, 712, 714) based on said parameter values for said scan parameters (138) in said cells (502-516, 802-812, 910-912).

2. The ultrasound system (100, 200) of Claim 1, further comprising a beamformer (110) utilizes said scan parameters (138) during different stages of a patient examination in accordance with a patient protocol.

3. The ultrasound system (100, 200) of Claim 1, wherein said probe (106, 202) acquires (308) said ultrasound images (906) along both of said first and second scan planes (210, 712, 714) without moving said probe (106, 202).

4. The ultrasound system (100, 200) of Claim 1, wherein said memory (136) record a series of consecutive ultrasound images (906) acquired (308) along each of said at least two scan planes (210, 712, 714) for playback as a cine-loop (908).

5. The ultrasound system (100, 200) of Claim 1, wherein said scan parameters (138) include at least one of gain, depth, width, mode, zoom, rotation and tilt (816).

6. The ultrasound system (100, 200) of Claim 1, further comprising a processor (116, 118, 120, 122) for automatically calculating said parameter values for a second cell (502-516, 802-812, 910-912) in said template (140) based on said parameter values for a first cell (502-516, 802-812, 910-912) in said template (140) that are entered (304) by a user.

7. The ultrasound system (100, 200) of Claim 1, wherein said probe (106, 202) scans (306) the object at least first and second times substantially simultaneously along first and second scan planes (210, 712, 714) without moving the probe (106, 202) between said at least first and second times and without a user entering (304) new scan parameters (138).

8. The ultrasound system (100, 200) of Claim 1, wherein said scan parameters (138) include protocol generic parameters, said system including a processor (116, 118, 120, 122) automatically entering (304) said parameter values for said protocol generic parameters to said cells (502-516, 802-812, 910-912) of said template (140).

9. The ultrasound system (100, 200) of Claim 1, wherein said scan parameters (138) define at least three scan planes (210, 712, 714) intersecting along a common axis extending from said probe (106, 202), said probe (106, 202) scanning (306) the object substantially simultaneously along said at least three scan planes (210, 712, 714) based on said parameter values of an associated said cell (502-516, 802-812, 910-912).

10. A protocol-based ultrasound method (300), comprising:
providing (302) a template (140) comprised of cells (502-516, 802-812, 910-912), each of said cells (502-516, 802-812, 910-912) containing scan parameters (138) defining (304) a scan sequence for acquisition (308) of ultrasound images (906) along a scan plane (210, 712, 714) through an object;
prior to scanning (306) the object, entering (304) parameter values (138) for said scan parameters (138) associated with said cells (502-516, 802-812, 910-912) in said template (140) to define (304) said scan sequences along at least two scan planes (210, 712, 714); and
scanning (306) the object with an ultrasound probe (106, 202) to automatically and successively acquire (308) ultrasound images (906) along at least two scan planes (210, 712, 714) based on said parameter values for said scan parameters (138) in said cells (502-516, 802-812, 910-912).
